(19) Europäisches Patentamt
European Patent Office
Office européen des brevets

(11) **EP 2 777 491 A1**

(12) **EUROPÄISCHE PATENTANMELDUNG**

(43) Veröffentlichungstag:
**17.09.2014 Patentblatt 2014/38**

(51) Int Cl.:
*A61B 5/024* *(2006.01)*    *A61B 5/1455* *(2006.01)*

(21) Anmeldenummer: **13158577.0**

(22) Anmeldetag: **11.03.2013**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Senspec GmbH**
**18069 Rostock (DE)**

(72) Erfinder: **Kulcke, Axel**
**18233 Am Salzhaff (DE)**

(74) Vertreter: **Hepp Wenger Ryffel AG**
**Friedtalweg 5**
**9500 Wil (CH)**

(54) **Verfahren und Vorrichtung zur Überwachung von Vitalfunktionen**

(57)    Verfahren zur Überwachung von Vitalparametern eines Lebewesens, wobei breitbandiges Licht in das Gewebe eines Lebewesens eingestrahlt wird, ein Spektrum des eingestrahlten Lichtes aufgenommen wird und eine Auswertung der Absorption durchgeführt wird, wobei wenigstens ein erster Parameter auf der Basis der Absorptionswerte eines ersten Spektralbereiches und wenigstens ein zweiter Parameter auf der Basis der Absorptionswerte eines zweiten Spektralbereiches (13) ermittelt wird, der erste Parameter mit dem zweiten Parameter verglichen wird und anhand des Vergleichs wenigstens ein Vitalparameter bestimmt wird.

FIG. 3

**Beschreibung**

Die vorliegende Erfindung betrifft ein Verfahren und eine Vorrichtung zur Überwachung von Vitalfunktionen von Patienten.

Aus dem Stand der Technik sind verschiedenen Verfahren und Geräte bekannt, um die Vitalfunktionen von Patienten aufzunehmen und/oder zu überwachen. Insbesondere bei Notfällen, während der Erstvorsorgung, bei Operationen oder auch für ein Langzeitmonitoring oder eine Schlafüberwachung werden bevorzugt nichtinvasive Geräte und Verfahren eingesetzt. Derartige Geräte und Verfahren sollen schnelle und genaue Resultate liefern, um möglichst schnell über den Gesundheitszustand eines Patienten informiert zu sein oder Entscheidungen über weitere Massnahmen zu treffen.

Aus dem Stand der Technik sind beispielsweise Pulsoximeter bekannt, die die Sauerstoffsättigung von Blut messen. Typischerweise wird Licht in ein Gewebe emittiert und die Absorbtion gemessen. Beispielsweise wird ein Ohrläppchen oder ein Finger in einem Halter eines derartigen Gerätes aufgenommen. Die Messungen werden sowohl im roten als auch im nahen infraroten Bereich des Lichtes durchgeführt, typischerweise wird jeweils eine Messung bei einer Wellenlänge von 660nm und bei 940nm durchgeführt.

Ausserdem sind Pulsspektrometer bekannt, die geeignet sind, eine amplitudenbasierte Auswertung des Pulses mittels Spektroskopie vorzunehmen, wie z.B. in der WO 2011/161102 A1 offenbart.

Ein verbreitetes Problem derartiger Verfahren und Geräte besteht darin, dass Messwerte ungenau sind oder dass die Messwerte oder Ergebnisse verfälscht sind. Da die Messungen bei einzelnen Wellenlängenbereichen durchgeführt werden, sind sie äusserst empfindlich auf Veränderungen, zum Beispiel von Umgebungsbedingungen des Messobjekts. Ausserdem ist eine derartige amplitudenbasierte Auswertung anfällig auf Bewegungsartefakte. Bewegt sich ein Patient, kann beispielsweise das Blut beschleunigt oder abgebremst werden oder ein am Körper angebrachter Sensor kann verrutschen. Ausserdem besteht die Gefahr von Fremdeinflüssen, insbesondere von elektrischen Geräten, magnetischen oder elektrischen Feldern oder Spannungsschwankungen innerhalb der Geräte. Je schlechter die Vitalfunktionen eines Patienten sind, desto schwieriger ist es, genaue Resultate zu erhalten, da die Nebengeräusche, sogenannte Artefakte, im Verhältnis sehr gross werden.

Es existieren unterschiedliche Ansätze, derartige Bewegungsartefakte aus dem Messsignal auszuschliessen. Die US 2011/0245654 offenbart beispielsweise ein Verfahren, welches mit einem adaptiven Filter die Bewegungsartefakte herauszufiltern versucht. Nahes Infrarotlicht (NIR) sowie Rotlicht werden appliziert und die optische Dichte gemessen. In einem numerischen Verfahren werden gemessene Werte mit Vergleichswerten verglichen und die Sauerstoffsättigung des Blutes mittels der maximalen Ausschläge bestimmt. Dieses Verfahren ist aufwändig und kompliziert und verlangt eine hohe Rechenleistung.

Es ist daher eine Aufgabe der Erfindung, die Nachteile des Standes der Technik zu überwinden. Insbesondere soll ein Verfahren und eine Vorrichtung bereitgestellt werden, welche komplizierte Rechenvorgänge vermindert, auf Bewegungsartefakte weniger anfällig ist und somit Fehlalarme vermeidet.

Diese Aufgabe wird durch die in den unabhängigen Patentansprüchen definierten Verfahren und Vorrichtungen gelöst. Weitere Ausführungsformen ergeben sich aus den abhängigen Patentansprüchen.

Das erfindungsgemässe Verfahren ist zur Überwachung von Vitalparametern eines Lebewesens vorgesehen. Ein Vitalparameter ist insbesondere die Pulsrate oder die Sauerstoffsättigung des Blutes. Es ist aber auch vorstellbar, das Verfahren zur Überwachung weiterer Parameter wie Blutzucker oder anderen chemischen Bestandteilen des Blutes oder anderer Flüssigkeiten einzusetzen. Das Verfahren umfasst die folgenden Schritte. Breitbandiges Licht, bevorzugt gepulstes Licht, wird in Gewebe eines Lebewesens eingestrahlt. Dabei umfasst das Licht bevorzugt Wellenlängen in einem Spektralbereich von 400nm bis 850nm. Ein Spektrum des aus dem Gewebe zurückgegebenen Lichtes wird aufgenommen. Dabei kann das Licht im Gewebe entweder reflektiert oder transmittiert worden sein. Abschliessend wird eine Auswertung des aufgenommenen Spektrums durchgeführt. Zur Auswertung der Absorption wird wenigstens ein erster Parameter auf der Basis eines ersten Spektralbereichs innerhalb des Spektrums ermittelt. Ausserdem wird wenigstens ein zweiter Parameter auf der Basis wenigstens eines zweiten Spektralbereichs ermittelt. Der zweite Spektralbereich ist grösser als der erste Spektralbereich und enthält den ersten Spektralbereich zumindest teilweise. Der erste Parameter wird mit dem zweiten Parameter verglichen und wenigstens ein Vitalparameter anhand des Vergleichs bestimmt.

Sowohl oxigeniertes Hämoglobin (Hb02) als auch reduziertes Hämoglobin (HHb) weist in diesem Wellenbereich mehrere isosbestische Punkte auf. Insbesondere im Wellenbereich von 500nm bis 600nm befinden sich derartige Punkte. In diesem Bereich sind die Absorptionskoeffizienten des Hämoglobins höher und der relative Anteil zwischen Absorption des Hämoglobins und Streuung des Lichtes im Gewebe wird erhöht. Durch den Puls wird sowohl der Anteil des arteriellen Blutes im Gewebe erhöht, bzw. kontinuierlich verändert, als auch die Sauerstoffsättigung im Gewebe. Eine Grundabsorbtion durch die nicht oder nur langsam veränderlichen Bestandteile des Gewebes, beispielsweise Haut und Knochen, bleibt jedoch bestehen.

Die Spektren zeigen typischerweise die Absorption des eingestrahlten Lichtes im Blut und Gewebe. Der Parameter, der aus dem ersten, kleineren Spektralbereich ermittelt wird, erfährt deutliche Schwankungen in Abhängigkeit des Pulses, bzw. der Sauerstoffsättigung im Blut, insbesondere wenn der Bereich die Wellenlängen mit erhöhter Absorption des oxigenierten Hämoglobins Hb02 zwischen zwei isosbestischen Punkten umfasst. Der zweite Parameter, der aus einem breiteren Spektralbereich gewonnen wird stellt eine Basis dar, die nur geringen und/oder langsamen Änderungen unterworfen ist. Durch Vergleich dieser beiden Parameter kann ein Wert bestimmt werden, der beispielsweise eine Aussage über die Sättigung des Blutes mit Sauerstoff erlaubt. Erfolgt die Auswertung über einen breiten Spektralbereich mit mehreren Wellenlängen, ist der Einfluss der jeweiligen Veränderung einzelner Anteile geringer. Mit dem erfindungsgemässen Verfahren wird also nicht rein amplitudenbasiert gemessen. Vielmehr wird eine amplitudenbasierte Messung in einem Teilbereich des Spektrums durch eine Messung über einen breiteren Bereich normiert. Der Teilbereich betrifft dabei Wellenlängen, in denen die Absorption sich in Abhängigkeit der Sauerstoffsättigung in einer gleichen Weise ändert, typischerweise bei höherer Sättigung zunimmt. Der zweite Bereich umfasst Abschnitte, in denen die Absorption bei höherer Sauerstoffsättigung zunimmt und Abschnitte, in denen die Absorbtion bei höherer Sättigung abnimmt. Der gemessene Parameter des zweiten Bereiches spiegelt daher stärker die Streuung im Gewebe oder die Absorption im nicht oxigenierten Blut wieder als die Messung im ersten Bereich. Die Messung über den grösseren Bereich ist daher in einem weit geringerem Masse von der Sauerstoffsättigung abhängig als die Messung über den ersten, engeren Bereich. Durch Vergleich dieser Werte lässt sich eine sauerstoffbasierte statt einer amplitudenbasierten Messung erzielen.

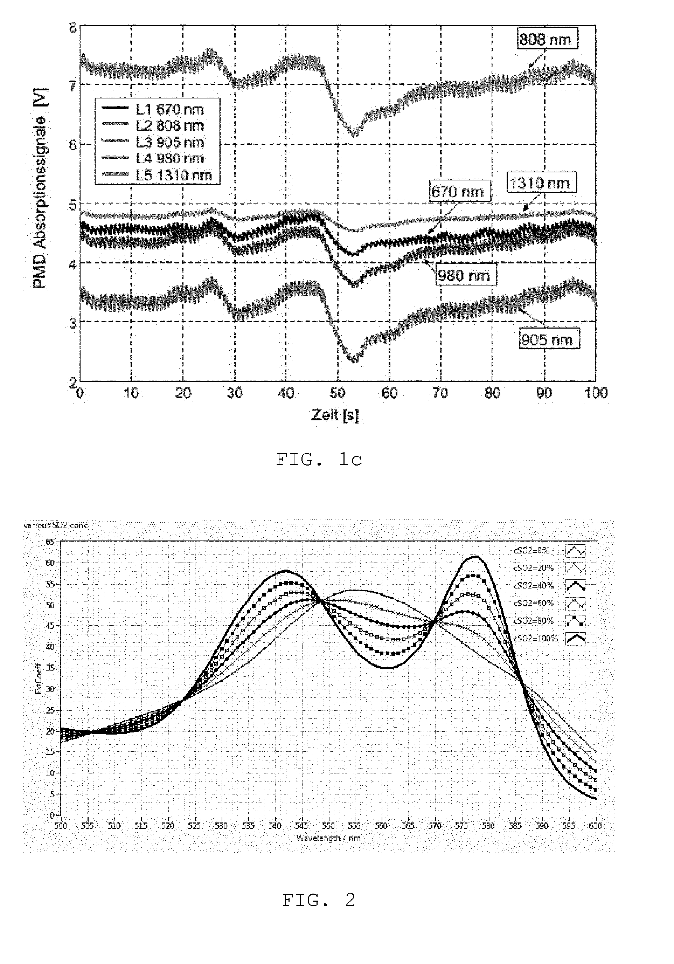

Bevorzugt ist der erste Bereich so gewählt, dass die Absorption bei längerer Sauerstoffsättigung zunimmt, das heisst, dass der Absorbtionskoeffizient Hb02 grösser ist, als der Absorptionskoeffizient HHb. In einer bevorzugten Ausführungsform entspricht der erste Spektralbereich im Wesentlichen einem Bereich zwischen mindestens zwei benachbarten isosbestischen Punkten, in denen sich die Absorption in Abhängigkeit der Sauerstoffsättigung gleich verhält, zum Beispiel zunimmt. Isosbestische Punkte befinden sich beispielsweise bei 505nm, 522nm, 548nm, 570 nm und 586nm (siehe dazu Fig. 2).

Der erste Spektralbereich kann aus zumindest zwei Teilbereichen zusammengesetzt sein. Bevorzugt weist jeder dieser Teilbereiche Absorptionskoeffizienten auf, die sich in Abhängigkeit der Sauerstoffsättigung gleich verhalten, zum Beispiel zunehmen. Werden die Teilbereiche entsprechend gewählt, summiert sich die Absorbtion. Somit kann auch bei geringer Sauerstoffsättigung eine Aussage gemacht werden.

Eine besonders vorteilhafte Möglichkeit des Vergleichs des ersten Parameters mit dem zweiten Parameter ergibt sich aus der Bildung eines Quotienten aus dem ersten und dem zweiten Parameter. Ein derartiger Quotient kann beliebig skaliert werden und stellt eine dimensionslose Grösse dar. Durch die Bildung eines Quotienten wird eine Grundabsorbtion, beispielsweise durch die Hämoglobinabsorption, Absorption der Haut, des Gewebes oder von Knochen verursacht, gefiltert, bzw. aus der Messung ausgeschlossen.

Der zweite Spektralbereich kann zumindest einen doppelt so grossen Wellenlängenbereich umfassen wie der erste Spektralbereich. Durch eine derartige breite Messung des zweiten Spektralbereiches wird ein Wert erreicht, der in Abhängigkeit von der Sauerstoffsättigung nur geringen Schwankungen unterworfen ist oder sich nur langsam verändert.

Bevorzugt umfasst der zweite Spektralbereich einen Wellenbereich, der mehrere isosbestische Punkte aufweist. Dies begünstigt einen Wert, der geringeren Schwankungen unterworfen ist.

Sowohl der erste als auch der zweite Parameter kann durch ein Integral der Absorptionskurve über den ersten bzw. den zweiten Bereich bestimmt werden. Durch die Integration ergeben sich einzelne Werte, die auf einfache Weise miteinander verknüpft werden können.

Bevorzugt wird mit dem erfindungsgemässen Verfahren ein Sauerstoffsignal erzeugt, welches die Auswertung zeitaufgelöst abbildet. Eine derartige Abbildung kann mit einer Frequenz von 50 bis 150 Hz aufgezeichnet werden. Damit kann die Sauerstoffsättigung über die Zeit abgebildet werden. Es ist dadurch beispielsweise möglich, eine Hypoxie zu erkennen. Sauerstoffwerte im Gewebe nehmen ab, die Vergleichswerte erfahren eine deutliche Änderung, da beispielsweise die Grundabsorption von einem längerfristigen Wert abweicht.

Es ist auch möglich, aus dem Sauerstoffsignal die Pulsrate zu bestimmen, da die Sauerstoffsättigung im Gewebe direkt vom Puls, bzw. vom frisch geförderten arteriellen d.h. sauerstoffangereicherten Blut beeinflusst wird. Somit kann sowohl eine Aussage über die Sauerstoffsättigung als auch über den Puls gemacht werden. Eine Kombination der Auswertung in einem Anzeigegerät oder die Weiterverarbeitung der Werte in Abhängigkeit zueinander ist nun möglich.

Der Pulsanteil in der Absorption macht nur einen geringen Teil, bei einem gesunden Menschen ca. 1% bis 5%, des gesamten optischen Signales aus. Bei kritischen Patienten kann dieser Anteil bis um den Faktor 10 oder mehr abnehmen. Durch das erfindungsgemässe Verfahren kann jedoch auch ein Puls bzw. eine Sauerstoffsättigung erfasst werden, die als kritisch einzustufen ist, bzw. ein Puls kann aus der Veränderung der Sauerstoffsättigung errechnet werden. Dies ist gemäss vorliegendem Verfahren möglich, da nicht das Absolutsignal ausgewertet wird, sondern bei Bildung eines Quotienten das Verhältnis der unterschiedlichen Absorption der einzelnen Wellen der jeweiligen Spektralbereiche.

Das Resultat des Vergleichs von dem ersten Parameter mit dem zweiten Parameter, welches beispielsweise als Vitalparameter vorliegen kann, kann mit einem rein amplitudenbasierten Signal verglichen werden. Es ist vorstellbar, dass ein derartiges Signal mittels der Absorption von eingestrahltem Licht bestimmt wird, wobei die Absorption beispielsweise bei einer bestimmten Wellenlänge gemessen wird. Gängige Wellenlängen aus dem Stand der Technik sind beispielsweise 660nm oder 940nm für die Messung der Sauerstoffsättigung des Blutes, andere Wellenlängen für andere Eigenschaften des Blutes sind vorstellbar und dem Fachmann bekannt. Typischerweise wird für die Generierung eines amplitudenbasierten Signales eine Wellenlänge oder ein Bereich des eingestrahlten Lichtes gewählt, der mehrere Wellenlängen umfasst, beispielsweise der Wellenbereich zwischen zwei isosbestischen Punkten. Ein derartiges Signal kann beispielsweise aus dem ersten oder zweiten gemäss dem vorliegenden Verfahren ermittelten Parameter gewonnen werden. Es ist aber auch vorstellbar, den Vergleich mit einem zweiten, unabhängigen amplitudenbasierten Signal durchzuführen, beispielsweise mit dem Signal eines Pulsoximeters.

Mit der erfindungsgemässen Lösung stehen ein sauerstoffbasiertes Signal und ein amplitudenbasiertes Signal zur Verfügung. Aus einem Vergleich dieser beiden Signale, bzw. aus dem Grad der Übereinstimmung dieser Signale kann ein Mass der Vertrauenswürdigkeit der Signale bestimmt werden. Es lassen sich so beispielsweise Alarme setzen, die bei sich ändernder Reaktion zwischen den Signalen ein Indiz sind, dass beispielsweise das eine Signal nicht mehr richtig erfasst wird, beispielsweise wenn das Licht nicht mehr richtig in das Gewebe eingestrahlt wird. Es ist auch vorstellbar, diese beiden Signale zeitbasiert aufzuzeichnen. Damit können auch längerfristige Änderungen oder Abweichungen ermittelt werden oder verschiedene Mittelwerte, insbesondere auch Mittelwerte über einzelne Intervalle oder Zeitabschnitte, miteinander verglichen werden.

Ein weiterer Aspekt der Erfindung betrifft eine Vorrichtung zur Überwachung von Vitalparametern eines Lebewesens, insbesondere zur Überwachung des Pulses. Bevorzugt eignet sich die Vorrichtung zur Durchführung des vorliegend beschriebenen Verfahrens. Eine derartige Vorrichtung umfasst zumindest eine Lichtquelle zum Einstrahlen von Licht in Gewebe. Die Lichtquelle emittiert bevorzugt ein breitbandiges Lichtspektrum. Insbesondere umfasst das Spektrum Licht mit einer Wellenlänge im Bereich von 400nm bis 850nm. Des Weiteren weist die Vorrichtung einen Empfänger für die Lichtstrahlung aus dem Gewebe auf. Der Empfänger umfasst ausserdem Mittel zur Auffächerung des Lichtes sowie einen Detektor, so dass ein Lichtspektrum des aus dem Gewebe zurückgegeben Lichtes detektierbar ist. Die Vorrichtung weist Auswertungsmittel auf. Diese Auswertungsmittel sind dazu geeignet, innerhalb des Spektrums wenigstens einen ersten Parameter auf der Basis eines ersten Spektralbereichs und wenigstens einen zweiten Parameter auf der Basis wenigstens eines zweiten Spektralbereichs zu ermitteln. Der zweite Spektralbereich ist grösser als der erste Spektralbereich. Ausserdem sind die Auswertungsmittel dazu geeignet, einen Vergleich von dem ersten Parameter mit dem zweiten Parameter durchzuführen und anhand des Vergleichs eine Bestimmung eines Vitalparameters durchzuführen. Dies begünstigt die Verarbeitung des Vitalparameters, beziehungsweise dessen Auswertung.

Bevorzugt umfassen die Auswertungsmittel Mittel zum Integrieren zumindest des ersten und des zweiten Spektralbereichs. Dadurch sind zumindest der erste sowie der zweite Parameter bestimmbar. Derartige bestimmte Werte können beispielsweise in einem Prozessor der Messvorrichtung weiterverarbeitet werden.

In einer bevorzugten Ausführungsform umfassen die Auswertemittel Mittel zur Bildung eines Quotienten aus dem ersten und dem zweiten Parameter. Als dimensionslose Grösse ist ein Quotient besonders vorteilhaft in der Auswertung bzw. Weiterverarbeitung. Mit der Bildung eines Quotienten kann eine Grundabsorption aus dem weiter zu verarbeitenden Signal ausgeschlossen werden. Somit werden nur Werte berücksichtigt, die sich verändern und dadurch von Interesse sind.

Ausserdem können die Auswertemittel Mittel zur Erzeugung eines Sauerstoffsignals umfassen, so dass die Auswertung zeitaufgelöst abbildbar ist. Eine derartige Abbildung über die Zeit lässt Rückschlüsse über den Verlauf des Vitalwertes zu. Insbesondere die zeitliche Änderung des Wertes ist ersichtlich.

Die Vorrichtung kann Befestigungsmittel aufweisen, bevorzugt Befestigungsmittel zum Befestigen der Vorrichtung an einem Finger, der Stirn, einem Ohrläppchen oder auf einer Hautoberfläche. Dies ist besonders vorteilhaft für den Fall, dass Messungen über einen längeren Zeitraum gemacht werden müssen.

Vorteilhaft umfasst der Lichtdetektor einen 2-dimensionales Sensorarray, insbesondere einen CMOS-Sensor, bevorzugt einen monolithischen CMOS-Pixelarray. Ein derartiger Sensor ermöglicht eine direkte Erfassung des Lichtspektrums, wobei beispielsweise jedem Bereich oder Pixel, bzw. jedem Pixelarray eine andere Wellenlänge zugeordnet ist und eine direkte Integration durch Aufaddieren von nebeneinanderliegenden Sensorzellen erlaubt. Ausserdem ist eine direkte Verstärkung und Weiterverarbeitung des Signales möglich.

Anhand von Figuren, welche lediglich Ausführungsbeispiele darstellen, wird die Erfindung im Folgenden näher erläutert. Es zeigen:

| | |
|---|---|
| Figur 1a: | Typische Messverläufe von Pulsoximetern aus dem Stand der Technik; |
| Figur 1b: | typische Rauschverhältnisse bei Blutmessungen in vitro; |
| Figur 1c: | typische reale Signale von Pulsoximetern; |
| Figur 2: | Absorptionsverlauf des Hämoglobins zwischen 500nm und 600nm; |
| Figur 3: | der Verlauf aus Figur 2 mit darin eingezeichneten Messbereichen; |

| Figur 4a: | ungestörte Pulskurve bei niedriger Frequenz; |
| Figur 4b: | ungestörte Pulskurve bei hoher Frequenz; |
| Figur 5a und 5b: | Pulskurven mit Störungen; |
| Figur 6 und 7: | Zwei Plethsignale über einen längeren Zeitraum; |
| Figur 8: | Frequenzspektrum des Pleth-Signals aus Figur 7; |
| Figur 9: | Darstellung einer Vorrichtung zur Durchführung des erfindungsgemässen Verfahrens; |
| Figur 10: | Schematische Darstellung der Vorrichtung aus Figur 9. |

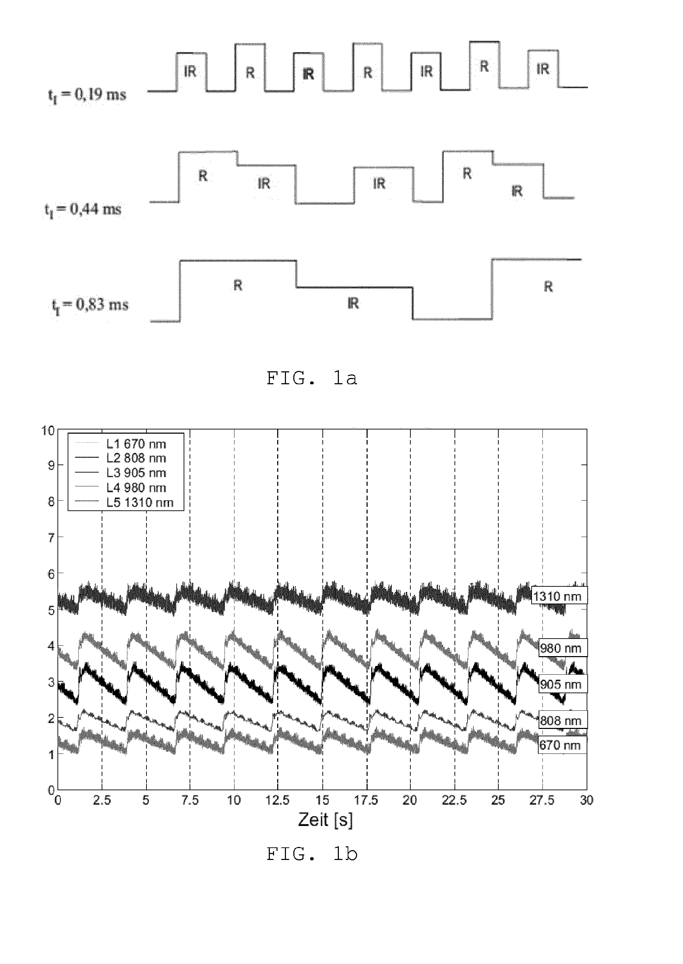

Figur 1a zeigt typische Sequenzen von Lichtaufnahmen von verschiedenen, auf dem Markt erhältlichen Pulsoximetern. Es gibt Geräte, die abwechslungsweise Infrarot- und Rotlicht aussenden, mit dazwischen liegenden Dunkelphasen (erste Kurve). Alternative Messgeräte zeigen hintereinandergeschaltete Verläufe (Kurve 3) oder Verläufe mit sich wiederholenden Einzelphasen (Kurve 2).

Figur 1b zeigt typische Rauschverhältnisse in technischen Aufbauten, beispielsweise eine Messung an einem Blutschlauch. Jedem dieser Graphen ist eine bestimmte Wellenlänge des Lichtspektrums zugeordnet. Allen gemein ist ein überlagertes Rauschen sowie eine pulsatile Änderung, hier verursacht durch eine Rollerpumpe.

Figur 1c zeigt nun für die gleichen Wellenlängen wie aus Figur 1b die realen Signale, wobei die Messung an einem gesunden Probanden durchgeführt wurde. Die Messung wurde am Mittelfinger des rechten Armes durchgeführt.

Figur 2 zeigt ein Absorptionsspektrum des Hämoglobins im Wellenbereich von 500nm bis 600nm für beispielhafte Sättigungen des Hämoglobins mit Sauerstoff zwischen 0% und 100%. Deutlich zu erkennen sind die isosbestischen Punkte bei 505nm, 522nm, 548nm, 570nm und bei 586nm. In einem isosbestischen Punkt wechselt der Zusammenhang von zunehmendem Absorptionskoeffizienten bei zunehmender Sauerstoffsättigung zu abnehmenden Absorptionskoeffizienten bei zunehmender Sauerstoffsättigung und umgekehrt. So ist der Absorptionskoeffizient beispielsweise der Kurven zwischen den isosbestischen Punkten bei 522nm und bei 548nm sowie der Kurven zwischen den isosbestischen Punkten bei 570nm und 586nm höher, je höher die Sauerstoffkonzentration ist.

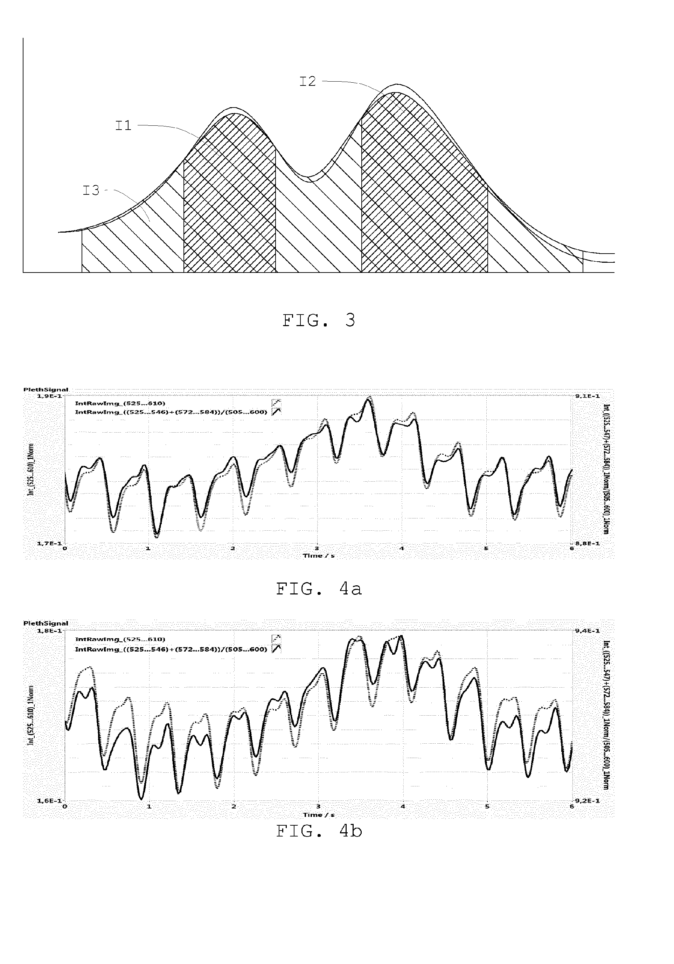

Figur 3 zeigt nun die ausgewählten Messbereiche für das vorliegende Verfahren. Die mit I1 und I2 bezeichneten Bereiche sind die Bereiche, in denen der Absorptionskoeffizient des sauerstoffgesättigten Blutes Hb02 höher ist, als jener des sauerstoffarmen Blutes HHb. Diese Bereiche bilden zusammen einen ersten Teilbereich des Absorptionsspektrums. Der mit I3 bezeichnete Bereich zeigt einen zweiten Bereich, der zur Ermittlung eines zweiten Parameters herangezogen wird. Beispielhaft dargestellt ist der Bereich zwischen 505nm und 600nm (vgl. dazu Figur 2) für einen Wert der Sauerstoffsättigung.

Die Kurven der Teilbereiche I1 und I2 werden jeweils separat zeitgleich oder versetzt integriert. Die Koeffizienten werden zu einem einzelnen Wert addiert. In einem weiteren Schritt, der gleichzeitig oder zeitversetzt ablaufen kann, wird die Kurve des zweiten Teilbereiches integriert. Ein gleichzeitiger Ablauf schliesst jedoch das serielle Abtasten der einzelnen Wellenlängen nicht aus. Aus den Werten dieser zwei Integrale kann ein Quotient gebildet werden, beispielhaft gezeigt in folgender Formel.

$$\frac{\int_{523}^{548} Absorbtion(f)df + \int_{570}^{586} Absorbtion(f)df}{\int_{505}^{600} Absorbtion(f)df} = P$$

I1 und I2 sind die Teilbereiche des ersten Spektralbereiches, I3 bezeichnet den zweiten Spektralbereich. P ist ein dimensionsloser Koeffizient, der zur Weiterverarbeitung verwendet werden kann.

Es ist selbstverständlich alternativ auch vorstellbar, dass die erste Messung in einem Teilbereich stattfindet, der tiefere Absorbtionskoeffizienten bei höherer Sauerstoffsättigung des Hämoglobins aufweist. Somit könnten weitere unterschiedliche Parameterverläufe generiert werden. Es ist beispielsweise auch vorstellbar, aus den Koeffizienten der Integration eine Differenz zu bilden.

Figur 4a zeigt beispielhaft die Gegenüberstellung dimensionsloser Koeffizienten mit einer amplitudenbasierten Messung eines niedrigen und ungestörten Pulses über die Zeit. Die wie im vorliegend beschriebenen Verfahren durchgeführten instantanen Messungen können beispielsweise mit einer Frequenz von 50 bis 150 Hz durchgeführt werden. Die so gewonnenen Werte ermöglichen es die Sauerstoffsättigung in Abhängigkeit der Zeit aufzuplotten und in einem Pleth-Diagramm darzustellen. Die Gegenüberstellung dieser sauerstoffbasierten Auswertung (als durchzogene Linie erkennbar) mit der amplitudenbasierten Messung (im vorliegenden Plot gestrichelt eingezeichnet) zeigt eine deutliche Übereinstimmung.

Figur 4b zeigt zu Figur 4a entsprechende Messresultate für einen schnellen Puls.

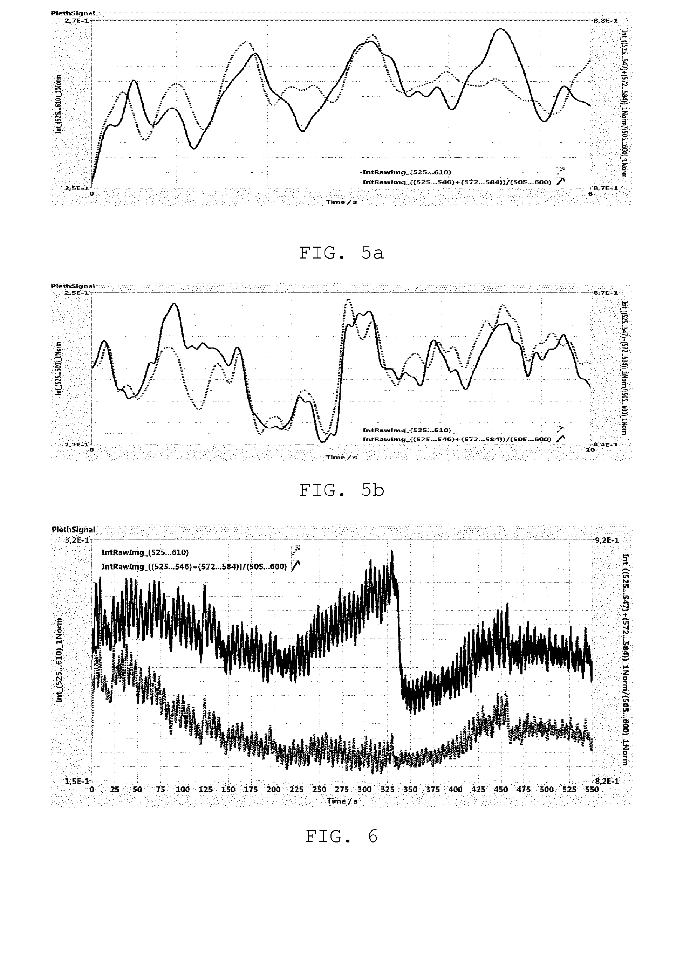

Figur 5a und 5b zeigen Pulskurven mit Störungen durch Bewegungsartefakte, wobei die Kurven jenen aus Figur 4a und 4b entsprechen. Die Ausprägung der Kurve, die aus dem dimensionslosen Koeffizienten gebildet wurde ist im Vergleich mit der amplitudenbasierten Messung ausgeprägter.

Figur 6 zeigt einen Pleth-Verlauf über einen längeren Zeitraum. Die deutlich sichtbare Frequenz ist die Atemfrequenz. Die Pulsfrequenz ist in dieser Auflösung nicht mehr darstellbar. Die amplitudenbasierte Kurve (gestrichelt dargestellt) weist keine auffälligen Merkmale auf. Hingegen zeigt die sauerstoffbasierte Auswertung (durchzogene Linie) eine deutliche Veränderung im Gegensatz zur amplitudenbasierten Auswertung. Ungefähr ab Sekunde 225 fangen die Signale der beiden Kurven an zu divergieren. Ungefähr ab Sekunde 310 fällt die sauerstoffbasierte Kurve markant ab. Das Verhalten im Bereich zwischen 200 und 300 Sekunden ist ein deutliches Zeichen für eine Hypoxie. Die Sauerstofunterversorgung wird durch das Ansteigen der Kurve angezeigt. Der markante Abfall der Kurve ungefähr ab Sekunde 310 zeigt eine schnelle Verbesserung der Sauerstoffversorgung.

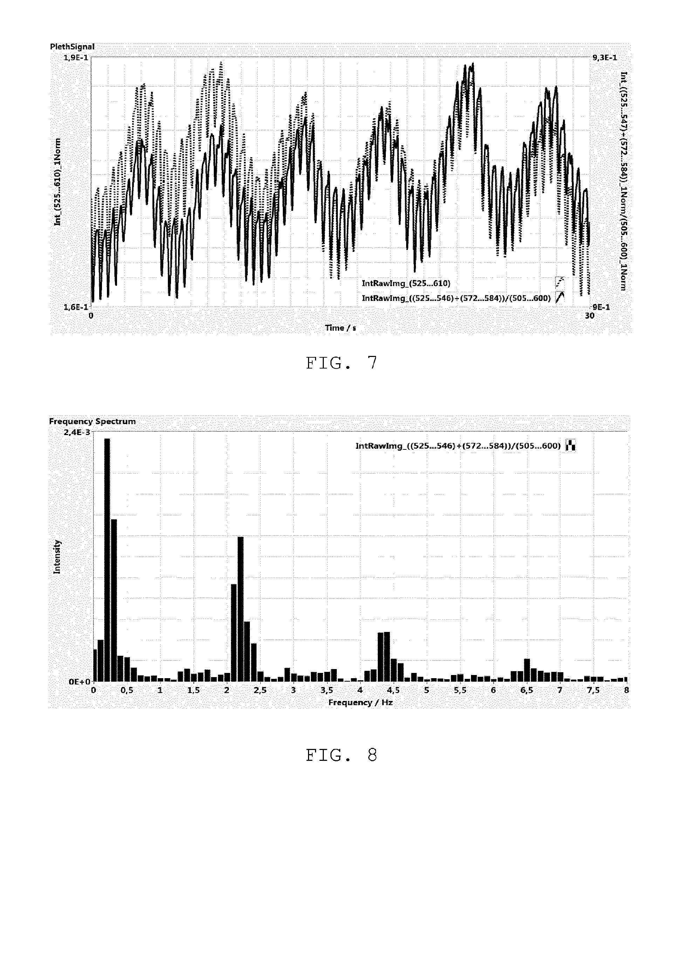

Die Pulssignale dieser beiden Kurven können ausserdem in einer feineren Auflösung, wie beispielsweise in Fig. 7 gezeigt, miteinander verglichen werden und somit gegeneinander verifiziert werden, da beide Signale einen in weiten Bereichen übereinstimmenden Pulsverlauf zeigen müssen. Störungen der einzelnen Messsignale sind somit detektierbar.

Figur 7 zeigt einen Ausschnitt aus einem Pleth-Verlauf mit einer höheren Auflösung und über einen kürzeren Zeitraum. Deutlich zu sehen ist der Puls. Die überlagerte niedere Frequenz ist die Atemfrequenz. Die Korrelation zwischen dem amplitudenbasierten (gestrichelt) und dem sauerstoffbasierten (durchgezogen) Signal ist eindeutig zu erkennen.

Eine Frequenzanalyse des sauerstoffbasierten, d.h. durchgezogen dargestellten Signals aus Figur 7 ist in Figur 8 dargestellt. Die Atemfrequenz liegt bei ca 0.2 Hz, die Pulsfrequenz bei ca. 2.2 Hz. Im Allgemeinen gilt bei Erwachsenen, dass die Atemfrequenz deutlich kleiner als die Pulsfrequenz ist. Die Pulsfrequenz ist ausserdem daran erkennbar, dass harmonische Oberfrequenzen erkennbar sind. Die Existenz derartiger Oberfrequenzen kann zur Verifikation der Pulsfrequenz herangezogen werden.

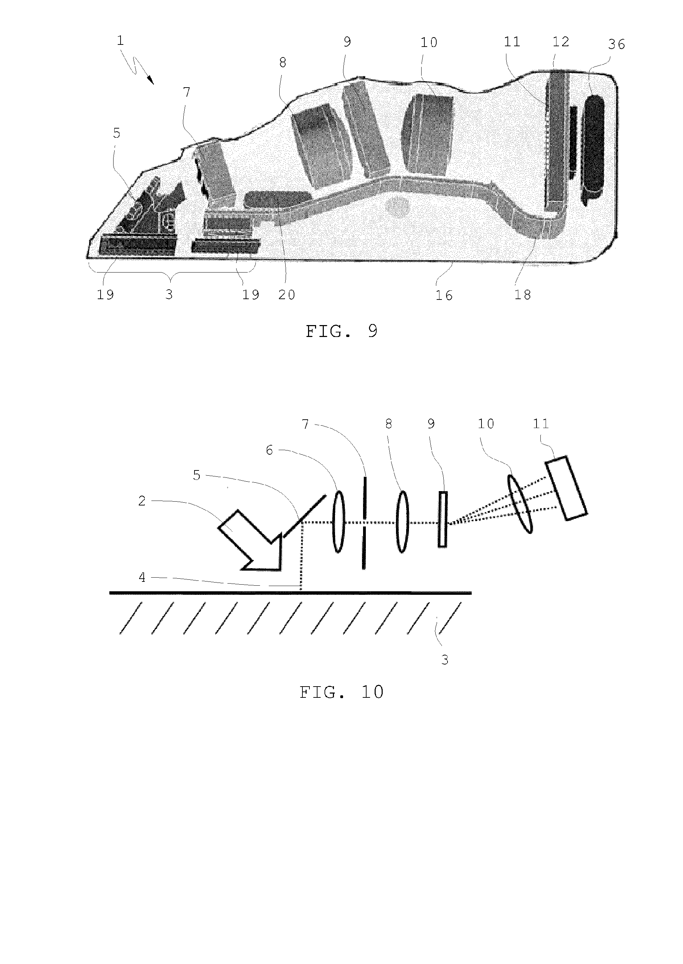

Figur 9 zeigt eine beispielhafte Ausführung einer erfindungsgemässen Vorrichtung, wie z.B in der WO2011/161102 zur Messung von Blutzucker beschrieben. Die Vorrichtung 1 weist ein Gehäuse auf, in dem die verschiedenen optischen und elektronischen Komponenten angeordnet sind. Die Messung erfolgt an einem Finger. Der Finger wird in den Messbereich 3 geführt oder an den Messbereich 3 herangeführt. Als Lichtquelle ist eine breitbandige LED 20 vorgesehen, die typischerweise Licht im Spektralbereich von 400 bis 850nm gegen den Messbereich 3 ausstrahlt. Das Gehäuse 16 weist eine Öffnung zum Austritt des Lichtes auf. Die Öffnung kann mit einer für das austretende Licht transparenten Abdeckung 19 versehen sein. Das durch den Finger reflektierte oder transmittierte Licht wird durch eine zweite Öffnung im Gehäuse 16, welche ebenfalls mit einer für das Licht transparenten Abdeckung 19 versehen ist, in das Gehäuse 16 geleitet. Zur Leitung und Auffächerung des Lichtes ist eine Spiegelanordnung 5, eine Spaltblende 7 und eine erste Abbildungsoptik 8 vorgesehen, welche das Licht auf ein Beugungsgitter 9 lenken. Durch das Beugungsgitter 9 wird das Licht wellenlängenabhängig aufgefächert und über eine zweite Abbildungsoptik 10 auf die Sensorfläche 11 eines Lichtdetektors, insbesondere eines Bildsensors 12 geleitet. Der Bildsensor 12 und die LED 20 sind auf einer gemeinsamen Leiterplatte 18 im Gehäuse 16 angeordnet. Die Leiterplatte 18 ist ausserdem mit Elektronikkomponenten zur Steuerung der LED 20 und des Bildsensors 12 versehen. Insbesondere weist die Leiterplatte 18 auch einen USB-Controller 36 und nicht genauer dargestellte USB Anschlüsse auf. Diese USB-Schnittstelle erlaubt einerseits Energiezufuhr zu der Vorrichtung 1. Andererseits ermöglicht sie Datenaustausch mit einem externen Rechner oder Anzeigegerät.

Figur 10 zeigt nun den schematischen Aufbau sowie die Funktionsweise der Anordnung aus Figur 9. Die Vorrichtung 1 weist eine oder mehrere Lichtquellen 2 (hier nur eine gezeigt) auf, welche Messlicht erzeugen. Die Lichtquelle 2 dient hierbei dazu, einen zu untersuchenden Messbereich 3, typischerweise einen Haut- und Gewebebereich, als im Wesentlichen zweidimensionalen Bereich mit relativ schmaler Erstreckung senkrecht zu seiner Oberfläche auszuleuchten. Der lineare Messbereich 3 wird somit in den verschiedenen Ausführungsformen jeweils durch die Beleuchtungseinrichtungen entweder reflexiv oder transmissiv beleuchtet und gibt Analyselicht 4 entsprechend seinem Transmissions- bzw. Reflexionsverhalten ab. Das Analyselicht 4 wird über einen Umlenkspiegel 5 in eine Spektrometereinheit zur Auffächerung des Lichts eingekoppelt, wobei eine derartige Spektrometereinheit zumindest eine Blende 7, eine erste Abbildungsoptik 8 und ein Beugungsgitter 9 umfasst. Das Analyselicht 4 liegt hierbei zur Bestimmung der Sauerstoffsättigung im Blut und weiterer Blutwerten im Wellenlängenbereich zwischen 400nm und 850nm und weist eine Spektralverteilung entsprechend der Substanzzusammensetzung auf. Somit enthält das Analyselicht Spektren im relevanten Wellenlängenbereich zur Identifizierung der quantitativen Substanzzusammensetzung im Messbereich 3, also typischerweise der Substanzzusammensetzung des arteriellen Blutes und des Gewebes.

Das Analyselicht 4 gelangt über einen Umlenkspiegel 5 und eine dritte Abbildungsoptik 6 auf eine Blende 7. Die dritte Abbildungsoptik 6 dient als Eingangsobjektiv für die Spektrometereinheit. Die Blende 7 ist länglich ausgebildet, vorzugsweise als Spalt oder Schlitz, z. B. mit einer Breite von typisch 10 μm bis 30 μm, und erstreckt sich in horizontaler Richtung bzw. z-Richtung (senkrecht zur Zeichenebene in Figur 10). In den Strahlengang können weitere optische Elemente wie z. B. Filter oder weitere Spiegel eingesetzt sein, um das Licht zu filtern oder zu lenken.

Der von der Blende 7 durchgelassene Streifen des Bildes des Messbereichs 3 wird als Licht über eine erste Abbildungsoptik 8 auf ein Beugungsgitter 9 geworfen. Das Gitter ist für Blutwertmessungen im Monitoring typischerweise ein transmissives "Volume Phase Holographic"-Gitter mit einer Blazewellenlänge im Bereich 500nm bis 800nm und ca. 300 1/mm bis 6001/mm. Das Gitter 9 ist so aufgebaut und angeordnet, dass eine wellenlängendispersive Auffächerung des Analyselichts 4 senkrecht zur Richtung des Spaltes der Blende 7 erfolgt, d. h. in Querrichtung bzw. Y-Richtung; hier sind entsprechend auch abgewandelte Ausführungsformen möglich. Über eine zweite Abbildungsoptik 10 wird das gebeugte Licht als Beugungsbild auf eine Sensorfläche 11 eines Bildsensors 12 abgebildet. Auf der Sensorfläche 11 wird somit ein Beugungsbild der Blende 7 bzw. deren Spalt abgebildet, mit der Längserstreckung des Spalts (der z-Richtung) in einer Richtung und der wellenlängendispersiven Auffächerung des Beugungsbildes in der anderen Richtung. Der Bildsensor ist für Blutwertmessungen im Monitoring typischerweise ein CMOS-Kamerasensor des Typs Aptina MT9m032 (1,6 Mpixel) oder MT9P031 (5 Mpix).

Eine derartige Abbildung ermöglicht durch einfaches Auslesen die Integration. Jede Zeile oder Spalte kann den Wert einer spezifischen Wellenlänge erfassen. Durch einfaches addieren der einzelnen optischen Werte wir die Integration vollzogen. Es können beispielsweise auch nur die Werte aus bevorzugten Bereichen des Sensors 12, beispielsweise mit bestimmten Wellenlängen, ausgelesen und addiert werden. Dieser optische Wert kann beispielsweise direkt am Sensor 12 digitalisiert werden. Elektrische Störungen können somit grösstenteils ausgeschlossen oder zumindest vermindert werden. Der so berechnete Wert kann in einen Prozessor zur Aufbereitung oder Weiterverarbeitung weitergeleitet werden.

Der Wert kann in dem Prozessor oder in weiteren Auswertemitteln ausgewertet werden, wobei der Prozessor in einem entsprechend programmierten Rechner sein kann. Es ist auch möglich, dass die Auswertemittel Teil der Vorrichtung 1 sind und sich vollständig darin befinden. Eine externe Anordnung ist vorstellbar. Diese Anordnung kann beispielsweise über Ausgabemittel wie beispielsweise einen Bildschirm oder eine akustische Anzeige verfügen.

**Patentansprüche**

1. Verfahren zur Überwachung von Vitalparametern eines Lebewesens, insbesondere zur Überwachung der Pulsrate und/oder Sauerstoffsättigung, umfassend die folgenden Schritte:

   - Einstrahlen von breitbandigem Licht, bevorzugt gepulstem Licht, in Gewebe eines Lebewesens, wobei das Licht bevorzugt Wellenlängen in einem Spektralbereich von 400nm bis 850nm umfasst;
   - Aufnehmen eines Spektrums des aus dem Gewebe zurückgestrahlten Lichts, wobei das Licht im Gewebe entweder reflektiert oder transmittiert worden ist;
   - Durchführen einer Auswertung des aufgenommenen Spektrums, **dadurch gekennzeichnet, dass** die Auswertung folgende Schritte umfasst:
   - Ermitteln wenigstens eines ersten Parameters auf der Basis eines ersten Spektralbereichs innerhalb des Spektrums;
   - Ermitteln wenigstens eines zweiten Parameters auf der Basis wenigstens eines zweiten Spektralbereichs (13), der grösser ist als der erste Spektralbereich und den ersten Spektralbereich zumindest teilweise enthält;
   - Vergleichen von dem ersten Parameter mit dem zweiten Parameter
   - Bestimmung wenigstens eines Vitalparameters anhand des Vergleichs.

2. Verfahren nach Anspruch 1, **dadurch gekennzeichnet, dass** der erste Spektralbereich im Wesentlichen einem Bereich zwischen mindestens zwei benachbarten isosbestischen Punkten entspricht, wobei in dem ersten Spektralbereich bevorzugt der Absorptionskoeffizient Hb02 grösser ist als der Absorptionskoeffizient HHb.

3. Verfahren nach Anspruch 2, **dadurch gekennzeichnet, dass** der erste Spektralbereich aus zumindest zwei Teilbereichen (I1, 12) zusammengesetzt wird, wobei bevorzugt in jedem Teilbereich (I1, 12) ein Absorptionskoeffizient Hb02 grösser ist als ein Absorptionskoeffizient HHb.

4. Verfahren nach einem der vorhergehenden Ansprüche, **dadurch gekennzeichnet, dass** der Vergleich des ersten Parameters mit dem zweiten Parameter der Bildung eines Quotienten aus dem ersten und zweiten Parameter entspricht.

5. Verfahren nach einem der vorhergehenden Ansprüche, **dadurch gekennzeichnet, dass** der zweite Spektralbereich (13) zumindest einen doppelt so grossen Wellenlängenbereich umfasst wie der erste Spektralbereich.

6. Verfahren nach einem der vorhergehenden Ansprüche, **dadurch gekennzeichnet, dass** der zweite Spektralbereich (13) einen Wellenbereich umfasst, der mehrere isosbestische Punkte aufweist.

7. Verfahren nach einem der vorhergehenden Ansprüche, **dadurch gekennzeichnet, dass** sowohl der erste als auch der zweite Parameter durch ein Integral der Absorption über den ersten bzw. den zweiten Bereich (13) bestimmt werden.

8. Verfahren nach einem der vorhergehenden Ansprüche, **dadurch gekennzeichnet, dass** ein Sauerstoffsignal erzeugt wird, welches die Auswertung zeitaufgelöst abbildet, bevorzugt mit einer Frequenz von 50-150 Hz.

9. Verfahren nach einem der vorhergehenden Ansprüche, **dadurch gekennzeichnet, dass** aus dem Sauerstoffsignal die Pulsrate bestimmt wird.

10. Verfahren nach einem der vorhergehenden Ansprüche, **dadurch gekennzeichnet, dass** ein Resultat des Vergleichs von dem ersten Parameter mit dem zweiten Parameter, insbesondere der wenigstens eine anhand des Vergleiches bestimmte Vitalparameter, mit einem amplitudenbasierten Signal, insbesondere einem Signal aufgrund der Absorption von eingestrahltem Licht einer bestimmten Wellenlänge oder eines bestimmten Wellenlängenbereiches, oder dem Signal eines Pulsoximeters, verglichen wird.

11. Vorrichtung (1) zur Überwachung von Vitalparametern eines Lebewesens, insbesondere zur Überwachung des Pulses, bevorzugt zur Durchführung eines Verfahrens gemäss den vorhergehenden Ansprüchen, umfassend zumindest eine Lichtquelle (2) zum Einstrahlen von Licht in Gewebe, bevorzugt eine breitbandige Lichtquelle (2), insbesondere eine Lichtquelle (2) mit einer Wellenlänge im Bereich von 400-850nm, einen Empfänger für die Lichtstrahlung aus dem Gewebe, wobei der Empfänger bevorzugt zumindest Mittel zur Auffächerung des Lichtes sowie einen Lichtdetektor umfasst, so dass ein Lichtspektrums des vom Gewebe zurückgeworfenen Lichtes detektierbar ist, **dadurch gekennzeichnet, dass** die Vorrichtung Auswertungsmittel aufweist, die dazu ausgebildet sind, wenigstens einen ersten Parameter auf der Basis eines ersten Spektralbereichs innerhalb des Spektrums zu ermitteln sowie wenigstens einen zweiten Parameter auf der Basis wenigstens eines zweiten Spektralbereichs (13) zu ermitteln, wobei der zweite Spektralbereich (13) grösser ist als der erste Spektralbereich und wobei die Auswertungsmittel dazu ausgebildet sind, einen Vergleich von dem ersten Parameter mit dem zweiten Parameter zur Bestimmung eines Vitalparameters anhand des Vergleichs durchzuführen.

12. Vorrichtung (1) gemäss Anspruch 11, **dadurch gekennzeichnet, dass** die Auswertemittel Mittel zum Integrieren zumindest des ersten und des zweiten Spektralbereichs (13) umfassen.

13. Vorrichtung (1) gemäss einem der Ansprüche 11 bis 12, **dadurch gekennzeichnet, dass** die Auswertemittel Mittel zur Bildung eines Quotienten aus dem ersten und dem zweiten Parameter umfassen.

14. Vorrichtung (1) gemäss einem der Ansprüche 11 bis 13, **dadurch gekennzeichnet, dass** die Auswertemittel Mittel zur Erzeugung eines Sauerstoffsignals umfassen, so dass die Auswertung zeitaufgelöst abbildbar ist.

15. Vorrichtung (1) gemäss einem der Ansprüche 11 bis 14, **dadurch gekennzeichnet, dass** die Vorrichtung (1) Befestigungsmittel aufweist, bevorzugt Befestigungsmittel zum Befestigen der Vorrichtung an einem Finger, einem Ohrläppchen oder auf einer Hautoberfläche.

16. Vorrichtung (1) gemäss einem der Ansprüche 11 bis 15, **dadurch gekennzeichnet, dass** der Lichtdetektor einen 2-dimensionales Sensorarray (12), insbesondere einen CMOS-Sensor umfasst, bevorzugt einen monolithischen CMOS-Pixelarray.

FIG. 1a

FIG. 1b

FIG. 1c

FIG. 2

FIG. 3

FIG. 4a

FIG. 4b

FIG. 5a

FIG. 5b

FIG. 6

**PlethSignal**

FIG. 7

**Frequency Spectrum**

FIG. 8

FIG. 9

FIG. 10

Europäisches
Patentamt

European
Patent Office

Office européen
des brevets

**EUROPÄISCHER RECHERCHENBERICHT**

Nummer der Anmeldung

EP 13 15 8577

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2011/137115 A1 (EYE02 SCAN LLC [US]; KHOOBEHI BAHRAM [US]; WAFAPOOR HUSSEIN [US]; EATO) 3. November 2011 (2011-11-03) * Absätze [0006], [0048], [0060] - [0065]; Abbildungen 9,20-22 * | 1-16 | INV. A61B5/024 A61B5/1455 |
| X | US 2008/255457 A1 (KHOOBEHI BAHRAM [US] ET AL) 16. Oktober 2008 (2008-10-16) * Absätze [0035] - [037,]; Abbildungen 1,2,4,8-11 * | 1-16 | |
| A,D | WO 2011/161102 A1 (SENSPEC GMBH [DE]; KULCKE AXEL [AT]) 29. Dezember 2011 (2011-12-29) * Seite 44, Zeile 19 - Seite 46, Zeile 22; Abbildung 1.a) * | 1-16 | |
| A | US 2005/101850 A1 (PARKER DAWOOD [GB]) 12. Mai 2005 (2005-05-12) * Absatz [0054]; Abbildungen 2a,2b * | 1-16 | |
| A | US 2005/119543 A1 (PARKER DAWOOD [GB] ET AL) 2. Juni 2005 (2005-06-02) * Absätze [0042], [0043]; Abbildungen 1-3 * | 1-16 | RECHERCHIERTE SACHGEBIETE (IPC) A61B |
| A | A. HOEFT ; H. KORB ; J. STEINMANN AND R. DEVIVIE: "In Vivo Measurement Of Blood Oxygen Saturation By Analysis Of Whole Blood Reflectance Spectra", PROC. SPIE 1067, OPTICAL FIBERS IN MEDICINE IV, 15. Juni 1989 (1989-06-15), Seiten 62-68, XP002712588, DOI: 10.1117/12.952102 * Seite 67 * | 1-16 | |

-/--

Der vorliegende Recherchenbericht wurde für alle Patentansprüche erstellt

| Recherchenort | Abschlußdatum der Recherche | Prüfer |
|---|---|---|
| Den Haag | 13. September 2013 | Mecking, Nikolai |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................
& : Mitglied der gleichen Patentfamilie, übereinstimmendes Dokument

EPO FORM 1503 03.82 (P04C03)

Europäisches Patentamt

European Patent Office

Office européen des brevets

**EUROPÄISCHER RECHERCHENBERICHT**

Nummer der Anmeldung

EP 13 15 8577

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 5 494 032 A (ROBINSON MARK R [US] ET AL) 27. Februar 1996 (1996-02-27) <br> * Spalte 6, Zeilen 30-57 * <br> * Spalte 13, Zeilen 5-10 * <br> * Spalte 20, Zeilen 55-66 * <br> * Abbildung 1 * <br> ----- | 1-16 | |
| | | | **RECHERCHIERTE SACHGEBIETE (IPC)** |

Der vorliegende Recherchenbericht wurde für alle Patentansprüche erstellt

| Recherchenort | Abschlußdatum der Recherche | Prüfer |
|---|---|---|
| Den Haag | 13. September 2013 | Mecking, Nikolai |

**ANHANG ZUM EUROPÄISCHEN RECHERCHENBERICHT
ÜBER DIE EUROPÄISCHE PATENTANMELDUNG NR.**

EP 13 15 8577

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-09-2013

| Im Recherchenbericht angeführtes Patentdokument | Datum der Veröffentlichung | Mitglied(er) der Patentfamilie | | Datum der Veröffentlichung |
|---|---|---|---|---|
| WO 2011137115 A1 | 03-11-2011 | US | 2013225951 A1 | 29-08-2013 |
| | | WO | 2011137115 A1 | 03-11-2011 |
| US 2008255457 A1 | 16-10-2008 | AU | 2005226667 A1 | 06-10-2005 |
| | | CA | 2559900 A1 | 06-10-2005 |
| | | EP | 1729644 A2 | 13-12-2006 |
| | | IL | 178129 A | 31-07-2012 |
| | | US | 2008255457 A1 | 16-10-2008 |
| | | WO | 2005092008 A2 | 06-10-2005 |
| WO 2011161102 A1 | 29-12-2011 | AU | 2011269068 A1 | 10-01-2013 |
| | | CA | 2800463 A1 | 29-12-2011 |
| | | CN | 102946794 A | 27-02-2013 |
| | | EP | 2584956 A1 | 01-05-2013 |
| | | US | 2013066172 A1 | 14-03-2013 |
| | | WO | 2011161102 A1 | 29-12-2011 |
| US 2005101850 A1 | 12-05-2005 | AU | 5180499 A | 06-03-2000 |
| | | EP | 1104254 A2 | 06-06-2001 |
| | | EP | 1598003 A2 | 23-11-2005 |
| | | US | 6842635 B1 | 11-01-2005 |
| | | US | 2005101850 A1 | 12-05-2005 |
| | | US | 2005148835 A1 | 07-07-2005 |
| | | WO | 0009004 A2 | 24-02-2000 |
| US 2005119543 A1 | 02-06-2005 | KEINE | | |
| US 5494032 A | 27-02-1996 | DE | 69227545 D1 | 17-12-1998 |
| | | DE | 69227545 T2 | 29-04-1999 |
| | | EP | 0522674 A2 | 13-01-1993 |
| | | JP | H05261088 A | 12-10-1993 |
| | | US | 5494032 A | 27-02-1996 |

EPO FORM P0461

Für nähere Einzelheiten zu diesem Anhang : siehe Amtsblatt des Europäischen Patentamts, Nr.12/82

**EP 2 777 491 A1**

**IN DER BESCHREIBUNG AUFGEFÜHRTE DOKUMENTE**

**In der Beschreibung aufgeführte Patentdokumente**

- WO 2011161102 A1
- US 20110245654 A
- WO 2011161102 A